# EUROPÄISCHE PATENTSCHRIFT

(11) **EP 0 355 443 B1**
(45) Veröffentlichungstag und Bekanntmachung des Hinweises auf die Patenterteilung: **03.11.1993**
(21) Anmeldenummer: 89113720.0
(22) Anmeldetag: 25.07.1989
(51) Int. Cl.: C07C 263/04, C07C 265/14

(54) **Kreislaufverfahren zur Herstellung von (cyclo)aliphatischen Diisocyanaten**
Cyclic process for the preparation of (cyclo)aliphatic diisocyanates
Procédé cyclique pour la préparation de diisocyanates (cyclo)aliphatiques

(30) Priorität: 18.08.1988 DE 3828033
(43) Veröffentlichungstag der Anmeldung: 28.02.1990
(73) Patentinhaber: HÜLS AKTIENGESELLSCHAFT, 45764 Marl (DE)
(72) Erfinder: Bohmholdt, Gerd, Dr., D-4370 Marl (DE); Disteldorf, Josef, Dr., D-4370 Marl (DE); Kirchner, Peter, D-4630 Bochum 1 (DE); Michalczak, Hans-Werner, D-4690 Herne 2 (DE)

(56) Entgegenhaltungen:
- EP-A- 0 054 817
- EP-A- 0 126 299
- EP-A- 0 126 300
- PATENT ABSTRACTS OF JAPAN, Band 6, Nr. 266 (C-142)(1144), 25. Dezember 1982; & JP-A-57158747

## Beschreibung

Die Erfindung betrifft ein verbessertes Verfahren zur phosgenfreien Herstellung von (cyclo)aliphatischen Diisocyanaten durch Umwandlung von (cyclo)aliphatischen Diaminen in die entsprechenden Biscarbamate und deren kontinuierliche thermische Spaltung in der Flüssigphase ohne Lösungsmittel, wobei die bei der Spaltung entstehenden Nebenprodukte ausgeschleust und in die Biscarbamatherstellung zurückgeführt werden.

Eine Methode zur Herstellung von (cyclo)aliphatischen Biscarbamaten besteht in der Umsetzung von (cyclo)aliphatischen Diaminen mit Harnstoff und Alkoholen unter Abtrennung von Ammoniak, wie in EP-PS 18 586 beschrieben. Weitere Veröffentlichungen befassen sich mit der vollständigen oder teilweisen Substitution von Harnstoff oder Diaminen durch Carbonylgruppen-haltige Verbindungen, beispielsweise durch N-unsubstituierte Carbamate und/oder Dialkylcarbonate bzw. mono- oder disubstituierte Harnstoffe oder Polyharnstoffe (vgl EP-PS 27 952, EP-PS 27 953, EP-PS 28 331, EP-OS 126 299 und EP-OS 126 300), wie sie auch als Zwischenprodukte bei der obengenannten Grundreaktion von Diaminen nit Harnstoff und Alkoholen auftreten.

Die thermische Spaltung von (cyclo)aliphatischen und insbesondere aromatischen Mono- und Biscarbamaten in die entsprechenden Isocyanate und Alkohole ist seit langem bekannt und kann sowohl in der Gasphase bei hohen Temperaturen wie auch bei relativ niedrigen Temperaturen in der Flüssigphase durchgeführt werden. Problematisch ist jedoch bei beiden Verfahrensweisen, daß durch die thermische Belastung grundsätzlich auch unerwünschte Nebenreaktionen stattfinden. Diese mindern nicht nur die Ausbeuten, sondern führen insbesondere zu verharzenden Nebenprodukten, die den Ablauf eines technischen Prozesses durch Belegungen und Verstopfungen in Reaktoren und Aufarbeitungseinrichtungen erheblich stören.

Es hat daher nicht an Vorschlägen gefehlt, durch gezielte chemische und verfahrenstechnische Maßnahmen Ausbeuteverbesserungen bzw. Reduzierungen der Nebenproduktbildung herbeizuführen. So werden in den DE-PS 1 022 222, DE-AS 19 44 719, US-PS 3 919 279 und DE-AS 26 35 490 Katalysatoren beschrieben, die die Spaltungsreaktion der Carbamate beschleunigen. Es handelt sich dabei um eine Vielzahl basischer, saurer sowie metallorganischer Verbindungen, die zwar die Ausbeuten an Isocyanaten gegenüber nicht-katalysierten Reaktionen wesentlich verbessern, jedoch die Bildung von Nebenprodukten nicht verhindern können. Dasselbe gilt auch für die zusätzliche Verwendung von inerten Lösungsmitteln, wie sie ebenfalls in US-PS 3 919 279 und DE-AS 26 35 490 empfohlen werden, um eine möglichst gleichmäßige Verteilung der zugeführten Wärme und des Katalysators im Reaktionsmedium zu gewährleisten.

Weiterhin ist aus der EP-PS 54 817 bekannt, daß sich Monocarbamate bei relativ niedrigen Temperaturen, vorzugsweise unter vermindertem Druck, gegebenenfalls in Gegenwart von Katalysatoren und/oder Stabilisatoren in guten Ausbeuten ohne den Einsatz von Lösungsmitteln spalten lassen, wobei die Spaltprodukte Isocyanat und Alkohol unter Sieden der Reaktionsmischung abdestillieren und durch fraktionierte Kondensation getrennt aufgefangen werden. Außerdem ist in den angeführten Beispielen eine Teilausschleusung des Reaktionsgemisches zur Abtrennung der bei der Spaltung gebildeten Nebenprodukte beschrieben. Eine evtl. Möglichkeit der Verwertung dieser Rückstände ist nicht erwähnt.

Dagegen wird in der jüngeren EP-PS 61 013 desselben Anmelders die thermische Spaltung von aromatischen und (cyclo)aliphatischen Biscarbamaten unter Zusatz vergleichbarer, in der EP-PS 54 817 genannter Katalysatoren und Hilfsmitteln wiederum in Gegenwart von Lösungsmitteln vorgenommen. Letztere dienen offensichtlich auch der Aufnahme von gebildeten, schwerflüchtigen Nebenprodukten, die dann nach Ausschleusung abgetrennt und verworfen werden. Die Verwendung von unter Rückfluß siedenden Lösungsmitteln führt grundsätzlich zu einer Reduzierung der Raum/Zeit-Ausbeute an Isocyanaten und erfordert einen zusätzlichen hohen Energieaufwand. Über diesen sowie über den Rückgewinnungsgrad des Lösungsmittels werden keine Angaben gemacht. Weiterhin werden Hilfsmittel verwendet, die unter den Reaktionsbedingungen flüchtig sind und zur Verunreinigung der Spaltprodukte führen. Besonders auffällig ist der auf das gebildete Diisocyanat bezogene hohe Rückstandsanteil der ebenso wie der niedrige Betriebsdruck eine wirtschaftliche und störungsfreie technische Fahrweise infrage stellt.

Die EP-PS 92 738 beschreibt die thermische Spaltung u. a. des cycloaliphatischen Biscarbamates 5-(Ethoxycarbonylamino)-1-(ethoxycarbonylaminomethyl)-1,3,3-trimethylcyclohexan, wobei dieses in Gegenwart eines hochsiedenden Lösungsmittels in flüssiger Form an der Innenwand eines Rohrreaktors entlanggeführt wird. Nachteilig bei diesem Verfahren sind die niedrige Ausbeute (51,8 %) und Selektivität (91,9%) für das entsprechende Diisocyanat. Ergebnisse einer kontinuierlichen Fahrweise unter Rückführung des rekombinierten oder teilweise gespaltenen Biscarbamates werden ebensowenig erbracht wie Angaben über die Aufarbeitung des die Nebenprodukte und Katalysator enthaltenden Lösungsmittels.

Zusammenfassend kann festgestellt werden, daß in den zitierten Druckschriften, egal ob sie sich auf die Spaltung der Biscarbamate allein beziehen oder deren Herstellung einschließen, keine Hinweise auf eine die Ausbeute verbessernde Verwertung der bei der Carbamatspaltung anfallenden z. T. hohen Rückstandsanteile gegeben sind, die außerdem bei der destillativen Abtrennung durch Zersetzung und Bildung harzartiger Verkrustungen zur Verunreinigung des Destillates sowie zu Verstopfungen der Anlageteile führen.

EP-OS 133 274 beschreibt den Umsatz von N-substituierten Allophansäure- und/oder Polyallophansäureestern mit Alkoholen in Abwesenheit oder Gegenwart von Katalysatoren bei Temperaturen von mindestens 160 °C zu Carbamaten. Hierbei handelt es sich allerdings ausschließlich um solche Allophanate, wie sie im Destillationssumpf der Reindestillation des bei der Carbamatspaltung anfallenden Roh-Isocyanates durch Reaktion von Isocyanatgruppen- mit Urethangruppen-haltigen Verbindungen gebildet werden können.

Nebenprodukte, die während der thermischen Spaltung insbesondere von Biscarbamaten im Spaltreaktor entstehen, sind jedoch ein Vielstoffgemisch aus u. a. substituierten höhermolekularen Uretdion-, Isocyanurat-, Allophanat-, Harnstoff-, Polyuret-, Carbodiimid-gruppenhaltigen unverdampfbaren Verbindungen. Dies geht auch daraus hervor, daß sie sich nur zu einem geringen Anteil und nicht vollständig, wie die Allophanate gemäß EP-OS 133 274 mit Alkoholen zu Carbamaten umsetzen lassen.

Aufgabe der vorliegenden Erfindung war es, (cyclo)aliphatische Diisocyanate durch thermische Spaltung von entsprechenden Biscarbamaten nach einem technisch praktikablen Kreislaufverfahren kostengünstig und in hohen Ausbeuten unter Vermeidung der bekannten Nachteile herzustellen.

Überraschenderweise wurde nun gefunden, daß man die bei der thermischen Spaltung von Biscarbamaten entstehenden höhermolekularen Nebenprodukte, die zur Gewährleistung einer störungsfreien und selektiven Reaktion möglichst kontinuierlich aus dem Reaktor ausgeschleust werden müssen, in Gegenwart von Diaminen, Harnstoff und Alkohol zu einem erheblichen Teil wieder in Biscarbamate umwandeln und zurückführen kann. Der dann bei der Aufarbeitung der Biscarbamate verbleibende Rückstand ist relativ thermostabil und kann problemlos destillativ abgetrennt werden.

Gegenstand der vorliegenden Erfindung ist daher ein Kreislaufverfahren zur Herstellung von (cyclo)aliphatischen Diisocyanaten der Formel OCN-R¹-NCO durch Umwandlung von entsprechenden Diaminen in Biscarbamate und deren thermische Spaltung, welches dadurch gekennzeichnet ist, daß man
a) (cyclo)aliphatische Diamine der Formel

   H₂N-R¹-NH₂

   und Nebenprodukte aus der thermischen Spaltung mit Harnstoff und Alkoholen der Formel

   R²-OH

   in Gegenwart von N-unsubstituierten Carbamaten und Dialkylcarbonaten zu Biscarbamaten der Formel

   R²O-CO-NH-R¹-NH-CO-OR²

   unter gleichzeitiger Abtrennung des entstehenden Ammoniaks umsetzt, wobei in diesen Formeln
   - R¹: für einen geradkettigen oder verzweigten aliphatischen Kohlenwasserstoffrest mit insgesamt 4 bis 12 Kohlenstoffatomen oder einen gegebenenfalls substituierten cycloaliphatischen Kohlenwasserstoffrest mit insgesamt 5 bis 13 Kohlenstoffatomen,
   - R²: für einen Rest, wie er durch Entfernung der Hydroxylgruppe aus einem primären aliphatischen Alkohol mit 1 bis 8 Kohlenstoffatomen
   steht,
b) von nicht umgesetztem Alkohol, N-unsubstituiertem Carbamat und Dialkylcarbonat zur Rückführung in a) sowie unverwertbarem Rückstand destillativ abtrennt,
c) die kontinuierliche thermische Spaltung der Biscarbamate in der Flüssigphase ohne Lösungsmittel in Gegenwart von Katalysatoren unter Sieden des Reaktionsgemisches und Rektifikation der Dämpfe vornimmt,
d) Diisocyanat und Alkohol als Rohprodukte fraktioniert kondensiert und das Rohdiisocyanat einer Reindestillation unterwirft,
e) einen Teil des Reaktiongemisches der Spaltung mit den gebildeten Nebenprodukten kontinuierlich ausschleust und nach vorherigem Umsatz mit anfallendem Rohalkohol in a) zurückführt.

Zur Herstellung der Biscarbamate gemäß a) werden erfindungsgemäß Diamin und etwa zu 10 bis 50 Massen-% in Biscarbamat gelöste Nebenprodukte aus der thermischen Spaltung in einer Menge von 5 bis 80 g, bezogen auf 1 Mol Diamin, mit Harnstoff und Alkohol, wobei Diamin, Harnstoff und Alkohol im Molverhältnis 1 : 2,03 : 4,0 bis 1 : 2,2 : 10, vorzugsweise 1 : 2,06 : 7 bis 1 : 2,1 :7 vorliegen, in Gegenwart von N-unsubstituiertem Carbamat und Dialkylcarbonat in einer Menge von jeweils 1 bis 10 Mol-%, bezogen auf das Diamin, bei Temperaturen von 180 bis 250 °C, vorzugsweise 220 bis 240 °C und Drücken in Abhängigkeit vom verwendeten Alkohol von 2 bis 80 bar, vorzugsweise 10 bis 13 bar, innerhalb von 3 bis 20 Stunden, vorzugsweise 5 bis 8 Stunden, zur Reaktion gebracht. Die Herstellung der Biscarbamate kann sowohl diskontinuierlich als auch kontinuierlich, z. B. in einer Reaktorkaskade, erfolgen.

Als Diamine für das erfindungsgemäße Verfahren seien beispielsweise genannt:
aliphatische Diamine wie 1,4-Butandiamin, 2-Methyl-1,5-pentandiamin, 2-Ethyl-1,4-butandiamin, 1,6-Hexandiamin, 2,2,4(2,4,4)-Trimethyl-1,6-hexandiamin, 1,8-Octandiamin, 1,10-Decandiamin, 1,12-Dodecandiamin und cycloaliphatische Diamine wie 1,4-Cyclohexandiamin, 2(4)-Methyl-1,3-cyclohexandiamin, 1,3(4)-Cyclohexandimethanamin, 4,4′-Methylenbis(cyclohexanamin), 5-Amino-1,3,3-trimethyl-cyclohexanmethanamin, Octahydro-4,7-methano-1H-indendimethanamin.

Besonders bevorzugt sind 2-Methyl-1,5-pentandiamin, 2,2,4(2,4,4)-Trimethyl-1,6-hexandiamin und 5-Amino-1,3,3-trimethyl-cyclohexanmethanamin, im folgenden als MPDA, TMDA und IPDA bezeichnet.

Als Alkohole sind grundsätzlich alle primären aliphatischen Alkohole geeignet, die einerseits eine ausreichend große Differenz in der Siedetemperatur zum jeweiligen Diisocyanat aufweisen und andererseits ein Verdampfen des Biscarbamates und Kondensieren der Spaltprodukte unter verfahrenstechnisch günstigen Betriebsdrücken gestatten. Infrage kommen daher besonders Methanol, Ethanol, Propanol, Butanol, Isobutanol, Pentanol, Isopentanole, Hexanol, Isohexanole, 2-Ethylhexanol vorzugsweise Butanol.

Die in der Herstellung des Biscarbamates gemäß a) eingesetzten Nebenprodukte sind durch in der Praxis nicht vermeidbare Folge- und Nebenreaktionen bei der thermischen Spaltung von Biscarbamaten entstandene höhermolekulare, thermisch instabile Verbindungen, die in gelöster Form kontinuierlich mit einem Teil des Reaktionsgemisches ausgeschleust werden. Die Reaktorausschleusung wird nach Umsetzung freier NCO-Gruppen mit dem im Prozeß anfallenden Rohalkohol als 25 bis 50 %ige alkoholische Lösung von Nebenprodukten und Biscarbamaten im Massen-Verhältnis von etwa 1 : 9 bis 1 : 1 in die Herstellungsstufe gemäß a) zurückgeführt.

Bei den bereits erwähnten Einsatzprodukten N-unsubstituiertes Carbamat und Dialkylcarbonat handelt es sich um Zwischenprodukte, die in geringen Anteilen durch Reaktion von Alkohol mit Harnstoff entstehen, bei der destillativen Aufarbeitung des Biscarbamates als Vorlauf abgetrennt und in die Herstellungsstufe gemäß a) jeweils in einer Menge von 1 bis 10 Mol-%, bezogen auf das Diamin zurückgeführt werden.

Voraussetzung für einen möglichst quantitativen Umsatz von Diamin und Nebenprodukten ist neben einer ausreichend hohen Temperatur und Reaktionszeit die kontinuierliche und vollständige Entfernung des gebildeten Ammoniaks, das durch den unter Rückfluß siedenden Alkohol aus der Reaktionsmischung ausgetrieben wird. Die Kondensation des Alkohols erfolgt oberhalb 60°C, um gegebenenfalls geringfügig auftretende Belegungen durch Ammoniumcarbamat/-carbonat bzw. N-unsubstituiertes Carbamat zu vermeiden.

Die Aufarbeitung des Biscarbamates gemäß b) erfolgt durch Abdestillieren des überschüssigen Alkohols sowie anschließende Abtrennung eines Vorlaufs und des unverdampfbaren Rückstandes in Dünnschichtverdampfern unter reduziertem Druck. Der N-unsubstituiertes Carbamat und Dialkylcarbonat enthaltende Vorlauf wird in die Biscarbamat-Herstellung gemäß a) zurückgeführt, der nicht mehr verwertbare Rückstand verworfen.

Die kontinuierlich betriebene thermische Spaltung des Biscarbamates in Diisocyanat und Alkohol gemäß c) erfolgt in einem Rührreaktor in Gegenwart von Katalysatoren, vorzugsweise unter Vakuumbedingungen und Temperaturen, bei denen der Reaktorinhalt siedet. Das nicht oder nur partiell gespaltene Einsatzprodukt (Biscarbamat und Monoisocyanatomonocarbamat) fließt nach Rektifikation in den Reaktor zurück. Diisocyanat und Alkohol werden gemäß d) fraktioniert als Rohprodukte auskondensiert.

Die gebildeten Nebenprodukte werden durch kontinuierliches Ausschleusen eines Teils der Reaktionsmischung gemäß e) aus dem Reaktor entfernt, wobei das Mengenverhältnis der Reaktorausschleusung zu eingesetztem Biscarbamat 1 : 20 bis 1 : 1,5, vorzugsweise 1 : 8 bis 1 : 3, beträgt. Die ausgeschleuste Menge wird, abhängig vom herzustellenden Diisocyanat, so bemessen, daß sich eine hinreichend niedrige stationäre Nebenproduktkonzentration einstellt, wie sie für einen ungestörten und selektiven Reaktionsablauf notwendig ist.

Das Rohdiisocyanat wird einer Feindestillation unterworfen, wobei der neben restlichen Anteilen von Diisocyanat im wesentlichen Monoisocyanato-monocarbamat enthaltende Sumpf zusammen mit der Reaktorausschleusung und dem im Prozeß anfallenden und geringe Mengen Biscarbamat enthaltenden Rohalkohol nach Umsetzung der freien NCO-Gruppen in die Biscarbamat-Herstellung gemäß a) zurückgeführt wird.

Die thermische Spaltung der Biscarbamate wird in Gegenwart von Katalysatoren durchgeführt, insbesondere von solchen Verbindungen, welche einen katalytischen Einfluß auf Veresterungsreaktionen nehmen. Hierzu zählen tert. Amine, Lewis-Säuren, Carbonsäuresalze, Metalloxide und metallorganische Verbindungen. Bei der Auswahl der Katalysatoren ist zu beachten, daß diese aus Gründen der Produktreinheit und konstanter Spaltaktivität unter den geltenden Reaktionsbedingungen thermostabil und nicht flüchtig sind. Als Katalysatoren, welche diesen Anforderungen genügen, seien beispielhaft genannt: Tris(dodecyl)amin, Tris(octadecyl)amin, Eisen(II)-chlorid, Kupfer(I)-chlorid, Zinkchlorid, -bromid, -iodid, Cadmiumiodid, Zinn(II)-chlorid, -bromid, -iodid, Zinn-, Zink-, Eisen-, Cobalt-, Manganoctoat und -naphthenat, Kupfer(II)-oxid, Zinn(IV)-oxid, Mangan(IV)-oxid, Dibutylzinnoxid, Dibutylzinn-dilaurat und Titan(IV)-2-ethylhexanolat. Besonders bevorzugt sind Zinkchlorid, -bromid, -iodid, Zinn(II)-chlorid, -bromid und -iodid.

Die Katalysatoren werden zweckmäßigerweise in Konzentrationen von 0,0001 bis 10 Massen-%, vorzugsweise 0,0005 bis 0,05 Massen-% und insbesondere 0,001 bis 0,02 Massen-%, bezogen auf das zu spaltende Biscarbamat, eingesetzt.

Die folgenden Beispiele dienen der weiteren Erläuterung des erfindungsgemäßen Kreislaufverfahrens, ohne auf diese zu beschränken, wobei eine Versuchsanordnung gemäß dem dargestellten Blockschema benutzt wurde. In das insgesamt geschlossene Verfahren werden Diamine und Harnstoff eingespeist, Diisocyanate, Ammoniak und Rückstand unter Kreisen der übrigen Produktströme entnommen. Alle Prozentangaben beziehen sich, sofern nicht anders vermerkt, auf Massenprozente.

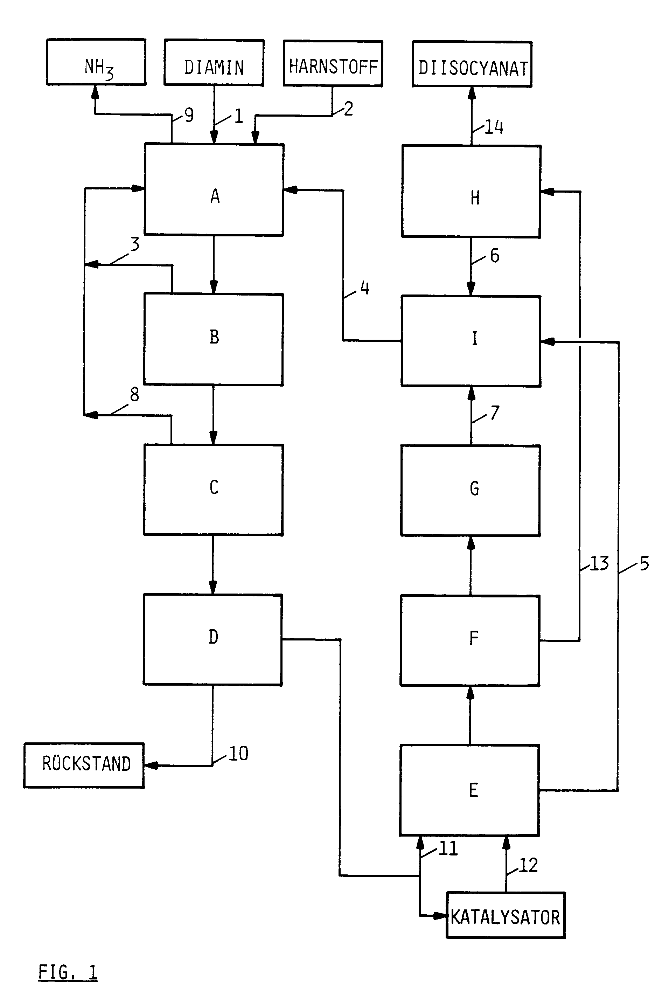

### Beispiel 1 (vgl. Fig. 1)

### a) Herstellung von 1,5-Bis(butoxycarbonylamino)-2-methylpentan (MPDU) aus MPDA, Nebenprodukten der thermischen Spaltung, Harnstoff und Butanol

In einem von zwei abwechselnd betriebenen ölbeheizten 25 l-Rührautoklaven mit nachgeschalteten Kondensatoren, beheizten Abgasleitungen und Entspannungsventilen (A) wurden 2 000 g MPDA (1) und eine Lösung (4), die durch Zusammenführen von 1 740 g Reaktorausschleusung (5) aus der thermischen Spaltung (E) mit ca. 30 % Nebenprodukten, 151 g Sumpf (6) aus der MPDI-Reindestillation (H) und 3 082 g Rohbutanol (7) aus der Alkohol-Kondensation (G) entstanden war, mit 2 131 g Harnstoff (2), 6 379 g Butanol (3), entsprechend einem Molverhältnis MPDA : Harnstoff : Butanol von 1 : 2,06 : 7 sowie 250 g Vorlauf (8) aus dem Dünnschichtverdampfer (C) der Biscarbamat-Aufarbeitung, der neben restlichen Mengen Butanol und MPDU etwa gleiche Teile Butylcarbamat und Dibutylcarbonat enthielt, innerhalb von ca. 2 h auf 230 °C aufgeheizt und weitere 5 h bei dieser Temperatur umgesetzt. Das gebildete Ammoniak (9) wurde bei Erreichen eines Druckes von ca. 12 bar laufend entspannt, wobei Butanol unter Rückfluß siedete. Die Kondensatortemperatur war auf 70 °C eingestellt, um Belegungen durch Butylcarbamat und gegebenenfalls Ammoniumcarbamat/-carbonat zu vermeiden.

Nach Beendigung der Reaktion wurde das im Überschuß verwendete Butanol in einer kontinuierlich bei 160 °C und 80 mbar betriebenen Kolonne (B) abdestilliert, wobei 6 379 g Butanol (3) erhalten wurden. Anschließend erfolgte in einem Dünnschichtverdampfer (C) bei 190 °C und 1 mbar die Abtrennung von 250 g eines Vorlaufes (8), der zusammen mit dem abgetriebenen Butanol (3) in den zweiten Autoklaven der MPDU-Herstellung (A) eingesetzt werden konnte. Das so gewonnene Roh-MPDU wurde in einem weiteren Dünnschichtverdampfer (D) bei 230 °C und 0,5 mbar vom unverdampfbaren, nicht mehr in MPDU überführbaren, schmelzflüssigen Rückstand (10) befreit. Die Menge an MPDU-Destillat betrug 7 722 g, an Rückstand 148 g.

### b) Thermische Spaltung von MPDU in 1,5-Diisocyanato-2-methylpentan (MPDI) und Butanol

Die Spaltung von MPDU erfolgte bei 233 °C und 27 mbar in Gegenwart von etwa 200 ppm Zinkchlorid als Katalysator in einem ölbeheizten 750 ml-Rührreaktor aus Stahl mit aufgesetzter Rektifiziersäule (E). Dieser wurde bei einem konstanten Reaktorinhalt von 200 g über eine Dosiereinrichtung gemäß der vorhandenen Spaltleistung kontinuierlich mit 598 g/h MPDU (11) in schmelzflüssiger Form (80 °C) beschickt.

Zur Abtrennung der entstehenden Nebenprodukte wurden 174 g/h des Reaktorinhalts ausgeschleust (5) sowie zur Aufrechterhaltung desselben und des Katalysatorpegels 174 g/h 200 ppm Zinkchlorid enthaltendes MPDU (12) über eine weitere Dosiereinrichtung eingespeist.

Die Spaltung erfolgte unter intensivem Sieden des Reaktionsgemisches, wobei die austretenden Dämpfe zur Abtrennung von MPDU und Monoisocyanato-monocarbamat in eine Rektifiziersäule gelangten, aus der sie in den Reaktor zurückflossen.
Die Spaltgase MPDI und Butanol wurden in zwei hintereinandergeschalteten Kondensatoren (F, G) bei 50 °C bzw. 10 °C auskondensiert. Das gewonnene, etwa 95 %ige Roh-MPDI (13) wurde einer Reindestillation (H) unterworfen, wobei 274,9 g/h MPDI (14) mit einer Reinheit > 99 % und 15,1 g/h eines im wesentlichen aus Monoisocyanato-monocarbamat bestehenden Sumpfes (6) anfielen. Die erhaltenen 308 g/h Roh-Butanol (7) mit etwa 5 % MPDU-Rekombinat wurden zusammen mit der Reaktorausschleusung (5) sowie dem Destillationssumpf (6) in dem Rührkessel (1) für die folgende MPDU-Herstellung gesammelt.

Die aus a) unter Rückführung der Nebenprodukte erhaltenen 7 722 g MPDU entsprechen bei einem Einsatz in die Spaltung von 772 g/h unter gleichzeitiger Ausschleusung eines Teils der Reaktionsmischung einem Intervall von 10,0 Stunden. Aus den während dieser Zeit gewonnenen 2 749 g MPDI errechnet sich eine Ausbeute bzw. Selektivität des Gesamtverfahrens von 94,9 %, bezogen auf eingesetztes MPDA, wobei diese auch über längere Laufzeiten der beiden Stufen a) (intermittierend) und b) (kontinuierlich) aufrechterhalten bleibt.

### Beispiel 2 (Vergleich)

### Thermische Spaltung von MPDU in MPDI und Butanol unter destillativer Abtrennung der Nebenprodukte aus der Reaktorausschleusung

Wie in Beispiel 1 b) beschrieben, wurden kontinuierlich über eine Zeit von 10 Stunden 772 g/h MPDU in den Reaktor zur Spaltung eingespeist und entsprechend 174 g/h des Reaktorinhalts ausgeschleust. Nach Reindestillation des Roh-MPDI wurden 2 751 g MPDI mit einer Reinheit von >99 % erhalten. Die Reaktorausschleusung von 1 740 g und 151 g Sumpf der Reindestillation wurden nach Umsetzung freier NCO-Gruppen mit 3 082 g Roh-Butanol in einem Dünnschichtverdampfer destilliert, wobei 1 160 g MPDU zurückgewonnen werden konnten. Hieraus errechnet sich eine Selektivität der Spaltung von 82,1 %. Der verbleibende harzartige Rückstand ließ sich nicht mehr in MPDU überführen.

### Beispiel 3 (Vergleich)

### Thermische Spaltung von MPDU in MPDI und Butanol und thermische Nachbehandlung der Reaktorausschleusung mit Butanol

Wie in Beispiel 1 b) beschrieben, wurden kontinuierlich über eine Zeit von 7,5 Stunden 772 g/h MPDU in den Reaktor zur Spaltung eingespeist und entsprechend 174 g/h des Reaktorinhalts ausgeschleust. Nach Reindestillation des Roh-MPDI wurden 2 062 g MPDI mit einer Reinheit von > 99 % erhalten. Die Reaktorausschleusung von 1 305 g und 113 g Sumpf der Reindestillation wurden mit 2 310 g Roh-Butanol im Autoklaven 5 Stunden bei 230 °C gerührt.

Die anschließende destillative Aufarbeitung im Dünnschichtverdampfer ergab 1 130 g MPDU und somit eine Gesamtselektivität für Spaltung und Nachbehandlung der Nebenprodukte von 86,5 %.

Anmerkung zu den Vergleichsbeispielen 2 und 3:
Bei der destillativen Abtrennung von Rückständen aus der thermischen Spaltung ohne oder mit vorheriger Nachbehandlung mit Alkohol (Alkoholyse) ergaben sich verfahrenstechnische Probleme durch das Auftreten nur schwer entfernbarer Anbackungen und Verkrustungen in der Destillationsapparatur, wie sie nach dem erfindungsgemäßen Verfahren nicht beobachtet wurden.

### Beispiel 4 (vgl. Fig. 1)

### a) Herstellung von 5-(Butoxycarbonylamino)-1-(butoxycarbonylaminomethyl)-1,3,3-trimethylcyclohexan (IPDU) aus IPDA, Nebenprodukten der thermischen Spaltung, Harnstoff und Butanol

Analog der in Beispiel 1 a) beschriebenen Verfahrensweise wurden 3 400 g IPDA (1) und eine Lösung (4), die durch das Zusammenführen von 1 350 g Reaktorausschleusung (5) aus der thermischen Spaltung (E) mit ca. 40 % Nebenprodukten, 207 g Sumpf (6) aus der IPDI-Reindestillation (H) und 3 446 g Rohbutanol (7) aus der Alkoholkondensation (G) entstanden war, mit 2 472 g Harnstoff (2), 7 400 g Butanol (3) sowie 300 g Vorlauf (8) aus dem Dünnschichtverdampfer (C) zur Umsetzung gebracht, wobei das Molverhältnis IPDA : Harnstoff : Butanol 1 : 2,06 : 7 betrug. Nach entsprechender Aufarbeitung wurden 9 296 g IPDU-Destillat und 146,5g Rückstand (10) erhalten.

### b) Thermische Spaltung von IPDU in 5-Isocyanato-1-(isocyanatomethyl-1,3,3-trimethylcyclohexan (IPDI) und Butanol

Die Spaltung erfolgte analog dem Beispiel 1 b) bei 235 °C und 27 mbar unter kontinuierlicher Zufuhr von jeweils 795 g/h IPDU (11) und 135 g/h 200 ppm Zinkchlorid enthaltendes IPDU (12) sowie Ausschleusung (5) von 135 g/h des Reaktorinhaltes. Das bei 50 °C auskondensierte Roh-IPDI (13) ergab nach Reindestillation 429,3 g/h IPDI (14) mit einer Reinheit von > 99 %. Die erhaltenen 345 g/h Rohbutanol (7) mit etwa 5 % IPDU-Rekombinat wurden zusammen mit der Reaktorausschleusung (5) sowie 20,7 g/h Destillationssumpf (6) in dem Rührkessel (I) für die folgende IPDU-Herstellung gesammelt.

Die aus a) unter Rückführung der Nebenprodukte erhaltenen 9 296 g IPDU entsprechen bei einem Einsatz in die Spaltung von 930 g/h unter gleichzeitiger Ausschleusung eines Teils der Reaktionsmischung einem Intervall von 10,0 Stunden. Aus den während dieser Zeit gewonnenen 4 293,5 g IPDI errechnet sich eine Ausbeute bzw. Selektivität des Gesamtverfahrens von 96,7 %, bezogen auf eingesetztes IPDA.

### Beispiel 5 (vgl. Fig. 1)

### a) Herstellung von 1,6-Bis(butoxycarbonylamino)-2,2,4(2,4,4)-trimethylhexan (TMDU) aus TMDA, Nebenprodukten der thermischen Spaltung, Harnstoff und Butanol

Analog der in Beispiel 1 a) beschriebenen Verfahrensweise wurden 2 850 g TMDA (1) und eine Lösung (4), die durch das Zusammenführen von 1 900 g Reaktorausschleusung (5) aus der thermischen Spaltung (E) mit ca. 30 % Nebenprodukten, 156 g Sumpf (6) aus der TMDI-Reindestillation (H) und 3 173 g Rohbutanol (7) aus der Alkoholkondensation (G) entstanden war, mit 2 229 g Harnstoff (2), 6 674 g Butanol (3) sowie 300 g Vorlauf (8) aus dem Dünnschichtverdampfer (C) zur Umsetzung gebracht, wobei das Molverhältnis von TMDA : Harnstoff : Alkohol 1 : 2,06 : 7 betrug. Nach entsprechender Aufarbeitung wurden 8 873 g TMDU-Destillat und 144 g Rückstand (10) erhalten.

### b) Thermische Spaltung von TMDU in 1,6-Diisocyanato-2,2,4(2,4,4)-trimethylhexan (TMDI) und Butanol

Die Spaltung erfolgte analog dem Beispiel 1 b) bei 238 °C und 27 mbar unter kontinuierlicher Zufuhr von jeweils 697 g/h TMDU (11) und 190 g/h 10 ppm Zinn(II)-chlorid enthaltendes TMDU (12) sowie Ausschleusung (5) von 190 g/h des Reaktorinhaltes. Das bei 50 °C auskondensierte Roh-TMDI (13) ergab nach Reindestillation 364,4 g/h TMDI (14) mit einer Reinheit von > 99 %. Die erhaltenen 317 g/h Rohbutanol (7) mit etwa 5 % TMDU-Rekombinat wurden zusammen mit der Reaktorausschleusung (5) sowie 15,6 g/h Destillationssumpf (6) in dem Rührkessel (I) für die folgende TMDU-Herstellung gesammelt.

Die aus a) unter Rückführung der Nebenprodukte erhaltenen 8 873 g TMDU entsprechen bei einem Einsatz in die Spaltung von 887 g/h unter gleichzeitiger Ausschleusung eines Teils der Reaktionsmimischung einem Intervall von 10,0 Stunden. Aus den während dieser Zeit gewonnenen 3 644 g TMDI errechnet sich eine Ausbeute bzw. Selektivität des Gesamtverfahrens von 96,2 %, bezogen auf eingesetztes TMDA.

## Patentansprüche

1. Kreislaufverfahren zur Herstellung von (cyclo)aliphatischen Diisocyanaten der Formel OCN-R¹-NCO durch Umwandlung von entsprechenden Diaminen in Biscarbamate und deren thermische Spaltung, dadurch gekennzeichnet, daß man
a) (cyclo)aliphatische Diamine der Formel
H₂N-R¹-NH₂
und Nebenprodukte aus der thermischen Spaltung mit Harnstoff und Alkoholen der Formel
R²-OH
in Gegenwart von N-unsubstituierten Carbamaten und Dialkylcarbonaten zu Biscarbamaten der Formel
R²O-CO-NH-R¹-NH-CO-OR²
unter gleichzeitiger Abtrennung des entstehenden Ammoniaks umsetzt, wobei in diesen Formeln
R¹ für einen geradkettigen oder verzweigten aliphatischen Kohlenwasserstoffrest mit insgesamt 4 bis 12 Kohlenstoffatomen oder einen gegebenenfalls substituierten cycloaliphatischen Kohlenwasserstoffrest mit insgesamt 5 bis 13 Kohlenstoffatomen,
R² für einen Rest, wie er durch Entfernung der Hydroxylgruppe aus einem primären aliphatischen Alkohol mit 1 bis 8 Kohlenstoffatomen
steht,
b) von nicht umgesetztem Alkohol, N-unsubstituiertem Carbamat und Dialkylcarbonat zur Rückführung in a) sowie unverwertbarem Rückstand destillativ abtrennt,
c) die kontinuierliche thermische Spaltung der Biscarbamate in der Flüssigphase ohne Lösungsmittel in Gegenwart von Katalysatoren unter Sieden des Reaktionsgemisches und Rektifikation der Dämpfe vornimmt,
d) Diisocyanat und Alkohol als Rohprodukte fraktioniert kondensiert und das Rohdiisocyanat einer Reindestillation unterwirft,
e) einen Teil des Reaktiongemisches der Spaltung mit den gebildeten Nebenprodukten kontinuierlich ausschleust und nach vorherigem Umsatz mit anfallendem Rohalkohol in a) zurückführt.

2. Kreislaufverfahren nach Anspruch 1,
dadurch gekennzeichnet,
daß die bei der thermischen Spaltung von Biscarbamaten c) als Nebenprodukte entstehenden höhermolekularen, thermisch instabilen Verbindungen mit einem Teil des Reaktionsgemisches gemäß e) ausgeschleust und in die Biscarbamat-Herstellung a) in Mengen von 5 bis 80 g, bezogen auf 1 Mol Diamin, eingesetzt werden.

3. Kreislaufverfahren nach Anspruch 1,
dadurch gekennzeichnet,
daß als (cyclo)aliphatische Diamine 1,4-Butandiamin, 2-Methyl-1,5-pentandiamin, 2-Ethyl-1,4-butandiamin, 1,6-Hexandiamin, 2,2,4(2,4,4)-Trimethyl-1,6-hexandiamin, 1,8-Octandiamin, 1,10-Decandiamin, 1,12-Dodecandiamin, 1,4-Cyclohexandiamin, 2(4)-Methyl-1,3-cyclohexandiamin, 1,3(4)-Cyclohexandimethanamin, 4,4′-Methylenbis(cyclohexanamin), 5-Amino-1,3,3-trimethylcyclohexanmethanamin, Octahydro-4,7-methano-1H-indendimethanamin in die Biscarbamat-Herstellung a) eingesetzt werden.

4. Kreislaufverfahren nach Anspruch 1,
dadurch gekennzeichnet,
daß als Alkohole Methanol, Ethanol, Propanol, Butanol, Isobutanol Pentanol, Isopentanole, Hexanol, Isohexanole, 2-Ethylhexanol in die Biscarbamat-Herstellung a) eingesetzt werden.

5. Kreislaufverfahren nach Anspruch 1,
dadurch gekennzeichnet,
daß das Molverhältnis von Diamin, Harnstoff und Alkohol in der Biscarbamat-Herstellung a) 1 : 2,03 : 4,0 bis 1 : 2,2 : 10, vorzugsweise 1 : 2,06 : 7 bis 1 : 2,1 : 7 beträgt.

6. Kreislaufverfahren nach Anspruch 1,
dadurch gekennzeichnet,
daß die Biscarbamat-Herstellung a) bei Temperaturen von 180 bis 250 °C, vorzugsweise bei 220 bis 240 °C und Drücken von 2 bis 80 bar, vorzugsweise 10 bis 13 bar, innerhalb von 3 bis 20 Stunden, vorzugsweise 5 bis 8 Stunden, durchgeführt wird.

7. Kreislaufverfahren nach Anspruch 1,
dadurch gekennzeichnet,
daß man die kontinuierliche thermische Spaltung c) der Biscarbamate bei Temperaturen von 180 bis 280 °C, vorzugsweise 230 bis 240 °C, und Drücken von 0,001 bis 2 bar, vorzugsweise 0,005 bis 0,05 bar, durchführt.

8. Kreislaufverfahren nach Anspruch 1,
dadurch gekennzeichnet,
daß man die thermische Spaltung c) der Biscarbamate in Gegenwart von Zinkchlorid, -bromid, -iodid, Zinn(II)-chlorid, -bromid oder -iodid als Katalysatoren vornimmt.

9. Kreislaufverfahren nach Anspruch 1,
dadurch gekennzeichnet,
daß in der thermischen Spaltung c) der Biscarbamate das Mengenverhältnis der Reaktorausschleusung zu eingesetztem Biscarbamat 1:20 bis 1:1,5, vorzugsweise 1:8 bis 1:3, beträgt.

10. Kreislaufverfahren nach Anspruch 1,
dadurch gekennzeichnet,
daß der Sumpf der Feindestillation nach Umsatz der freien NCO-Gruppen mit anfallendem Rohalkohol in die Biscarbamat-Herstellung a) zurückgeführt wird.

## Claims

1. Recycle process for the production of (cyclo)aliphatic diisocyanates of the formula OCN-R¹-NCO by the transformation of corresponding diamines into biscarbamates and their thermal splitting, characterized in that
a) (cyclo)aliphatic diamines of the formula
H₂N-R¹-NH₂
and by-products from the thermal splitting are reacted with urea and alcohols of the formula
R²-OH
in the presence of N-unsubstituted carbamates and dialkyl carbonates to biscarbamates of the formula
R²O-CO-NH-R¹-NH-CO-OR²
with simultaneous separation of the resulting ammonia, where in these formulae
R¹ stands for a straight-chained or branched aliphatic hydrocarbon radical with a total of 4 to 12 carbon atoms or an optionally substituted cycloaliphatic hydrocarbon radical with a total of 5 to 13 carbon atoms,
R² stands for a radical, such as one obtained by removing the hydroxyl group from a primary aliphatic alcohol with 1 to 8 carbon atoms,
b) there is removal by distillation from non-reacted alcohol, N-substituted carbamate and dialkyl carbonate for reintroduction into a) and unusable residue.
c) continuous thermal splitting of the biscarbamates is performed in the liquid phase without solvent in the presence of catalysts with boiling of the reaction mixture and rectification of the vapours,
d) diisocyanate and alcohol are fractionally condensed as crude products and the crude diisocyanate is subjected to fine distillation,
e) a part of the reaction mixture of the splitting is continuously washed-out with the formed by-products and reintroduced into a) after prior conversion with resulting raw alcohol.

2. Recycle process according to claim 1,
characterized in that
the high-molecular-weight, thermally unstable compounds forming as by-products during the thermal splitting of biscarbamates c) are washed out with a part of the reaction mixture according to e) and introduced into the biscarbamate production section a) in quantities of 5 to 80 g, relative to 1 mol diamine.

3. Recycle process according to claim 1,
characterized in that
1,4-butane diamine, 2-methyl-1,5-pentane diamine, 2-ethyl-1,4-butane diamine, 1,6-hexane diamine, 2,2,4-(2,4,4)-trimethyl-1,6-hexane diamine, 1,8-octane diamine, 1,10-decane diamine, 1,12-dodecane diamine, 1,4-cyclohexane diamine, 2(4)-methyl-1,3-cyclohexane diamine, 1,3(4)-cyclohexane dimethane amine, 4,4'-methylene-bis(cyclohexane amine), 5-amino-1,3,3-trimethylcyclohexane methane amine, octahydro-4,7-methano-1H-indendimethane amine are introduced into the biscarbamate production section a) as (cyclo)aliphatic diamines.

4. Recycle process according to claim 1,
characterized in that
methanol, ethanol, propanol, butanol, isobutanol, pentanol, isopentanols, hexanol, isohexanols, 2-ethylhexanol are introduced into the biscarbamate production section a) as alcohols.

5. Recycle process according to claim 1,
characterized in that
the molar ratio of diamine, urea and alcohol in the biscarbamate production section a) is 1: 2.03 : 4.0 to 1 : 2.2 : 10, preferably 1 : 2.06 : 7 to 1 : 2.1 : 7.

6. Recycle process according to claim 1,
characterized in that
biscarbamate production a) is carried out at temperatures of 180 to 250 °C, preferably at 220 to 240 °C and pressures of 2 to 80 bar, preferably 10 to 13 bar, within 3 to 20 hours, preferably 5 to 8 hours.

7. Recycle process according to claim 1,
characterized in that
the continuous thermal splitting c) of the biscarbamates is carried out at temperatures of 180 to 280 °C, preferably 230 to 240°C, and pressures of 0.001 to 2 bar, preferably 0.005 to 0.05 bar.

8. Recycle process according to claim 1,
characterized in that
the thermal splitting c) of the biscarbamates is carried out in the presence of zinc chloride, bromide, iodide, zinc(II) chloride, bromide or iodide as catalysts.

9. Recycle process according to claim 1,
characterized in that
in the thermal splitting c) of the biscarbamates, the quantity ratio of the reactor wash-out to biscarbamate used is 1:20 to 1:1.5, preferably 1:8 to 1:3.

10. Recycle process according to claim 1,
characterized in that
the bottom of the fine distillation is, after conversion of the free NCO groups with resulting raw alcohol, fed back into the biscarbamate production section a).

## Revendications

1. Procédé en circuit d'obtention de diisocyanates (cyclo)aliphatiques de formule OCN-R¹-NCO par transformation des diamines correspondantes en biscarbamates et par dissociation thermique de ceux-ci, caractérisé en ce que :
a) on fait réagir des diamines (cyclo)aliphatiques de formule
H₂N-R¹-NH₂
et les sous-produits provenant de la dissociation thermique avec de l'urée et des alcools de formule
R²-OH
en présence de carbamates non substitués à l'azote et de carbonates de dialcoyle, en biscarbamates de formule
R²O-CO-NH-R¹-NH-CO-OR²
avec élimination simultanée de l'ammoniac qui s'est formé,
formules dans lesquelles :
R¹ représente un radical hydrocarboné aliphatique linéaire ou ramifié, ayant au total de 4 à 12 atomes de carbone ou un radical hydrocarboné cycloaliphatique éventuellement substitué ayant au total de 5 à 13 atomes de carbone,
R² représente un radical comme il résulte de l'élimination d'un groupe hydroxyle à partir d'un alcool aliphatique primaire ayant de 1 à 8 atomes de carbone,
b) on sépare par distillation l'alcool qui n'a pas réagi, le carbamate non substitué à l'azote et le carbonate de dialcoyle pour retour à a) ainsi que le résidu non valorisable,
c) on effectue la dissociation thermique en continu du biscarbamate en phase liquide sans solvant, en présence de catalyseurs, à l'ébullition du mélange réactionnel et rectification des vapeurs.
d) on condense d'une manière fractionnée le diisocyanate et l'alcool en tant que produits bruts et on soumet le diisocyanate brut à une distillation de purification,
e) on évacue une partie du mélange réactionnel de la dissociation d'une manière continue avec les sous-produits formés et on recycle en a) après la conversion précédente avec de l'alcool brut résultant.

2. Procédé en circuit selon la revendication 1, caractérisé en ce que l'on évacue les composés instables thermiquement, à poids moléculaire élevé, qui se forment au cours de la dissociation thermique des biscarbamates c) en tant que sous-produits avec une partie du mélange réactionnel selon e) et que l'on met en oeuvre dans la production de biscarbamate a) en quantités allant de 5 à 80 g rapporté à 1 mol de diamine.

3. Procédé en circuit selon la revendication 1, caractérisé en ce que l'on met en oeuvre comme diamines (cyclo)aliphatiques, la 1,4-butanediamine, la 2-méthyl-1,5-pentanediamine, la 2-éthyl-1,4-butanediamine, la 1,6-hexanediamine, la 2,2,4-(2,4,4)-triméthyl-1,6-hexanediamine, la 1,8-octanediamine, la 1,10-decanediamine, la 1,12-dodécanediamine, la 1,4-cyclohexanediamine, la 2(4)-méthyl-1,3-cyclohexandiamine, la 1,3(4)cyclohexanedimethanamine, la 4,4'-méthylène-bis(cyclohexanamine), la 5-amino-1,3,3-triméthylcyclohexaneméthanamine, l'octahydro-4,7-méthano-1H-indènediméthanamine, dans la production des biscarbamates a).

4. Procédé en circuit selon la revendication 1, caractérisé en ce que l'on met en oeuvre dans la production de biscarbamate a) en tant qu'alcools, le méthanol, l'éthanol, le propanol, le butanol, l'isobutanol, le pentanol, les isopentanols, l'hexanol, les isohexanols, le 2-éthylhexanol.

5. Procédé en circuit selon la revendication 1, caractérisé en ce que le rapport molaire de diamine, d'urée et d'alcool dans la production de biscarbamate a) s'élève de 1 : 2,03 : 4,0 à 1 : 2,2 : 10, de préférence de 1 : 2,06 : 7 à 1 : 2,1 : 7.

6. Procédé en circuit selon la revendication 1, caractérisé en ce que l'on effectue la production des biscarbamates a) à des températures de 180 à 250°C, de préférence de 220 à 240°C, et à des pressions allant de 2 à 80 bar, de préférence de 10 à 13 bar, durant 3 à 20 heures - de préférence durant 5 à 8 heures.

7. Procédé en circuit selon la revendication 1, caractérisé en ce que l'on effectue la dissociation thermique en continu c) des biscarbamates à des températures allant de 180 à 280°C - de préférence de 230 à 240°C - et à des pressions allant de 0,001 à 2 bar - de préférence de 0,005 à 0,05 bar.

8. Procédé en circuit selon la revendication 1, caractérisé en ce qu'on entreprend la dissociation thermique c) des biscarbamates en présence de chlorure de zinc, de bromure de zinc, d'iodure de zinc, de chlorure stanneux (II), de bromure stanneux ou d'iodure stanneux comme catalyseurs.

9. Procédé en circuit selon la revendication 1, caractérisé en ce que dans la dissociation thermique c) des biscarbamates, le rapport pondéral de l'éclusage au dehors du réacteur au biscarbamate mis en jeu s'élève de 1 : 20 a 1 : 1,5 - de préférence de 1 : 8 à 1 : 3.

10. Procédé en circuit selon la revendication 1, caractérisé en ce que le résidu de la distillation fine après conversion des groupes NCO libres avec l'alcool brut qui s'est produit, est ramené dans la production de biscarbamate a).
